# EUROPEAN PATENT APPLICATION

(11) **EP 2 033 513 A1**
(43) Date of publication of application: **11.03.2009**
(21) Application number: 06764362.7
(22) Date of filing: 30.05.2006
(51) Int. Cl.: A01N 1/02, C12N 5/06

(54) **PROCEDURE FOR THE PREPARATION OF UNILAMELLAR VESICLES FOR CRYOPRESERVATION AND THE CULTURE OF STEM CELLS AND EMBRYOS**

(71) Applicant: Instituto Nacional De Investigacion y Tecnologia agraria y Alimentaria (INIA), 28040 Madrid (ES)
(72) Inventor: DE LA FUENTE MARTÍNEZ, Julio, 28040 Madrid (ES)
(74) Representative: Gonzalez Palmero, Fé
(86) International application number: PCT/ES2006/000310
(87) International publication number: WO 2007/138119

(57) **Abstract**

In order to prepare the unilamellar low-density lipid vesicles, a presolution of vegetable lipids is first prepared; a suitable quantity of lipids, between 4 and 12 mg/ml, mixed with pure water, heated and stirred for about 30 minutes; the suspension obtained is kept at 50-60°C for one hour and stirred for 2 minutes every 20 minutes. The high molecular weight hyaluronan is added to the lipidic suspension until a final concentration of 1 mg/ml is obtained, and the suspension of lipids and hyaluronan is emulsified by sonication, pumped using a homogenising valve and immediately made to go through a polycarbonate membrane, to obtain a final suspension with 70% of the particles in suspension having a size between 5 and 15 nm, which makes it possible to use the monolayer vesicles of HA and LDL for the cryopreservation and culture of different mammalian cells, specially germ cells.

## Description

### OBJECT OF THE INVENTION

This invention relates to a new method of preparing small unilamellar vesicles, specially conceived for the cryopreservation of spermatozoa, or preparing semen diluents, from different mammal species, for the culture and freezing of oocyte embryos produced *in vitro* and *in vivo.*

The object of the invention is, therefore, to provide a new method based on the use of unilamellar vesicles composed of high molecular weight hyaluronan (HA) molecules (5 to 15 nanometres in size), which have low-density lipids in suspension, to prepare semen diluents and obtain culture media for embryos of different mammal species.

Specifically, this invention is framed, like the main component of culture and freezing media for germ cells and mammalian embryos, within the science devoted to animal reproduction and the conservation of zoogenetic resources.

### BACKGROUND OF THE INVENTION

The currently existing methods for the cryopreservation of sperm cells is based on the use of egg yolk with buffered solutions that contain permeable cryoprotectors (glycerol, DMSO, etc.).

There are two major reasons why hen egg yolk is an essential component in the most widely used sperm diluents. Firstly, egg yolk contains a high concentration of low-density lipids (LDL), specifically, phosphatidylcholine (P-CH), which is the most common phospholipid in mammalian cell membranes, including sperm, oocyte and embryo membranes. Secondly, the lipids in hen egg yolk are very homogeneous vesicles that are less 50 nanometres (nm) in size.

The inefficiency in the incorporation into biological membranes is highly dependent on the size of the vesicles. The smaller the vesicles, the greater the incorporation of the sperm, the oocyte and the embryos through the membrane.

Mammals' bi-layer membranes are bound to each other by very weak Van der Waal's forces and, when the cells are withdrawn from the *in vivo* environment, they tend to lose phospholipids, specially from the membrane's outer layer, which is primarily composed of phosphatidylcholine and sphingomyelin. It has been proven that spermatozoa metabolise endogenous phospholipids during *in vitro* storage (Scoot and Dawson 1968).

Specifically, a significant decrease in phosphatidylcholine has been observed during the *in vitro* culture and storage of spermatozoa (Douard et al 2000). A similar reduction in lipids takes place during the handling or the culture of mammalian embryos (Palasz, unpublished observation). However, mammalian membranes may modify and carry exogenous lipids from the culture medium (Spector and Yorek 1985).

Phospholipid transfer has been successfully used to incorporate exogenous lipids into spermatozoa in humans (Arienti et al 1997), in bovines (Streiner and Graham 1997) and in porcines (He et al 2001).

However, the current preparation of lipidic vesicles is not very effective, since the vesicles produced are very heterogeneous. The current methods of preparing sperm diluents with different lipids from different sources produce vesicles with different sizes, which may range from 50 to 200 nm, and, therefore, are not homogeneous.

### DESCRIPTION OF THE INVENTION

The method of preparing unilamellar vesicles for the cryopreservation of spermatozoa from mammal species and for the culture of embryos produced *in vitro* and *in vivo* that the invention proposes solves, in a fully satisfactory manner, the problems described above, in regards to the different aspects discussed.

More specifically, it is a system that makes it possible to use small unilamellar vesicles composed of high molecular weight hyaluronan molecules (HA) which carry low-density lipids in suspension to prepare semen diluents and culture media for embryos of different mammal species.

The size reduction of the unilamellar vesicles is possible thanks to the use of a mixture of high molecular weight hyaluronidase, between 0.3 and 2 mg/ml, and low-density lipids, LDL, containing a high concentration of phosphatidylcholine, PCH, preferably of vegetable origin. This makes it possible to obtain unilamellar vesicles with a very small, homogeneous size, ranging between 5 and 15 nm. Moreover, this increases the efficiency of the incorporation of lipids into both sperm and embryo membranes, and, consequently, increases the efficiency of semen diluents and *in vitro* culture media for the embryos of different mammal species.

Hyaluronan (HA) is a simple polysaccharide with complex biological functions. Unlike other glycosaminoglycans, which are synthesised by the cytoplasm Golgi apparatus, HA is synthesised by the inner membrane of cell membranes. This shows that the interaction between phospholipids and HA begins very early in development and is crucial for the normal operation and development of mammalian cell membranes. The fact that HA is intracellularly present during the key moments of cell proliferation and migration suggests that HA is a key component for the intracellular regulation of these processes. Moreover, HA is a very effective antioxidant and, if used with materials of biological origin, such as egg yolk or bovine serum albumin (BSA), may very effectively prevent against the contamination of frozen samples by viruses, such as Newcastle disease (transmitted by egg yolk) or other pathogens which may be present in the samples.

Consequently, there is an immediate application for the use of the small vesicles composed of high molecular weight HA and LDL, preferably of vegetable origin, in the preparation of sperm diluents and *in vitro* culture media for embryos of different mammal species, both domestic and wild, also including humans.

In order to prepare hyaluronan (HA) and the unilamellar vesicles of low-density lipids, a presolution of vegetable lipids containing at least 10% phosphatidylcholine (PCH) is prepared; to this end, traditional stirring and mixing methods are used. A suitable quantity of lipids, e.g. between 4 and 12 mg/ml, is mixed with a small quantity of pure water, heated to 50-60°C, and constantly stirred with a stirrer, for example, a magnetic stirrer, for 30 minutes.

The suspension thus obtained is kept at 50-60°C for the next hour, and stirred for 2 minutes every 20 minutes. The high molecular weight hyaluronan is added to the lipidic suspension until a final concentration of 1 mg/ml is obtained.

The suspension of lipids and hyaluronan is emulsified by sonication (Sonicator, Sonic and Materials Inc., Danburry Connecticut, USA) in the following manner: at 10 seconds per pulse every 10 seconds with a pulse intensity of 30 units for 5 minutes. The lipidic suspension is pumped by a homogenising valve at 20,000 psi and, immediately thereafter, is made to go through a polycarbonate membrane 0.05 µ in diameter. In the final suspension produced, 70% of the particles (SUV) in suspension are within a range of between 5 and 15 nm.

The size and the distribution of the small unilamellar vesicles (SUV) were evaluated by specific fluorescent probe, Bodipy FL (Molecular Probes, Inc. Eugene OR, USA), and by means of a confocal microscope.

The two vesicle components, HA and LDL, are an integral, vital part of the spermatozoon's and the embryo's cell membranes, and are necessary for the normal biological function of cells under *in vitro* conditions, since there is a quick decrease in viability.

The use of these small vesicles, 5 to 15 nm in size, composed of HA and LDL considerably simplifies the preparation of diluents and facilitates the incorporation of compounds through the sperm and embryo cell membranes in *in vitro* culture systems, which entails a significant advantage.

Moreover, the use of the above-mentioned HA/LDL vesicles in sperm diluents and in *in vitro* culture media for embryos protects the frozen sperm cells and cultured embryos against any type of reactive oxygen (ROS) that is produced during the freezing-thawing and culture processes. The different types of radioactive oxygen (ROS) are amongst the main reasons for the low post-thaw survival rate of sperm cells and the low quality of embryos produced *in vitro.*

Another advantage derived from the use of HA and vegetable LDL vesicles is that they may eliminate the transmission potential of viruses and pathogens. Moreover, the use thereof makes it possible to freeze semen from wild species, which currently may not be frozen.

The HA/LDL vesicles, 5-15 nm in size, may be used with any of the permeable cryoprotectors that are currently most widely used, such as glycerol, DMSO, ethylene glycol, propanediol, and non-permeable cryoprotectors, such as different sugars or medium additives, such as antioxidants (EDTA), that are currently used in semen diluents and different culture media.

Finally, the use of LDL vesicles, alone or jointly with HA, for handling and culture may improve *in vitro* cultures and the viability of mammalian embryos after transfer or freezing.

### EMBODIMENT EXAMPLES OF THE INVENTION

The following examples illustrate the preferred embodiments of this invention, and are not at all intended to limit the scope thereof.

### Freezing of bull semen.

Semen from three crossed beef bulls was collected by artificial vagina. Ejaculates with a mininum 70% motility were divided into three equal parts and diluted at 26°C with each of the diluents to be tested:
- Diluent 1: (control) 1% milk fat containing fructose, citric acid (see the diluents' formulas), and supplemented with 8% intact egg yolk;
- Diluent 2: The complete egg yolk is replaced with rectified egg yolk at the same concentration (8%), by centrifugation at 3,000 x g for 1 hour at 5°C; and
- Diluent 3: the same as no. 2 plus 10 mg/ml of homogenised phospholipids containing 0.5% hyaluronan.

The ejaculates were divided into three equal parts, diluted at 26°C in each of the diluents and added until a final concentration of 7% glycerol was obtained. The semen was aspirated into 0.5-ml plastic straws, at 20 x 10⁶ sperm cells per straw, and frozen in vapours for 10 minutes, 7 cm away from the liquid nitrogen (LN₂), in order to be subsequently emptied into LN₂. The straws were thawed in a water bath at 37°C for 40 seconds.

Each experiment was replicated three times on different collection days. The sperm viability was measured by means of artificial insemination assays. The results were based on the pregnancy diagnoses made 45 days after the AI.

**Table 1. Pregnancy percentages in beef cows inseminated with semen frozen with Triladyl diluent with glycerol supplemented with rectified egg yolk and with low-density lipids (LDL) and high molecular weight hyaluronan (HA).**

| Bull | | Diluents used | | | | | |
|---|---|---|---|---|---|---|---|
| | | 1. With intact egg yolk | | 2. rectified egg yolk | | 3. LDL + Hyaluronan | |
| | | N of Cows AI | Pregnant % | N of Cows AI | Pregnant % | N of Cows AI | Pregnant % |
| | kA | | | | | | |
| | | 15 | 5^{a} (33.3) | 9/20 | 45.0^{b} | 11/22 | 50.0^{b} |
| | jB | | | | | | |
| | | 14 | 6 (42.8) | 8/19 | 42.1 | 9/20 | 45.0 |

| | | | | | | | |
|---|---|---|---|---|---|---|---|
| ^{a,b} *P* < 0.07 | | | | | | | |

### Results of the in vitro fertilisation

A total of 1,318 bovine oocytes were inseminated using the standardised *in vitro* fertilisation (IVF) method with semen frozen with Triladyl (n = 668) or in a homogenate and high molecular weight hyaluronan (L + HA; n = 650). The presumed zygotes were cultured in an SOFaa medium supplemented with BSA and HA. The division rate was evaluated 48 hours after the insemination and the number of blastocysts developed at 7 and 8 days of culture was determined. The oocytes inseminated with semen frozen in L + HA have a division rate (61.5%) and a rate of excluded blastocysts (46.9%) that is significantly higher (P<0.05) than for that frozen in Triladyl, 47.9% and 25.4%, respectively. These results may be observed in Table 2.

**Table 2. Fertilisation and division rates, number of bovine blastocysts produced with semen frozen in 2 different diluents: commercially used Triladyl or a diluent containing low-density lipids with high molecular weight hyaluronan (LDL + HA).**

| Diluent | Number of oocytes | Division rate (n, %) | Blastocysts (n, %) |
|---|---|---|---|
| Triladyl | 668 | 320 (47.9)^{a} | 170 (25.4)^{a} |
| HA + LDL | 650 | 400 (61.5)^{b} | 305 (46.9)^{b} |

| | | | |
|---|---|---|---|
| ^{a,b} *P* < 0.05 | | | |

### Freezing of equine stud semen

In a blind assay with three studs with different levels of fertility (high, medium and low), the sperm cells were frozen in 0.5-ml straws in three different diluents (in a 3 x 3 factorial design): A, German commercial diluent containing intact egg yolk (control); B, homogenate of lipids and high molecular weight HA to replace the egg yolk; and C, homogenate of lipids alone and to replace the egg yolk. The semen doses were frozen using the traditional slow protocol and thawed in a water bath at 39°C for 40 seconds; subsequently, the motility was evaluated in the standardised manner.

### Preparation of the semen

Immediately following the thawing, 0.5 ml of semen were transferred to the lower part of a sealed Pasteur pipette and covered, extremely carefully, with 0.4 ml of fert-TALP medium supplemented with: 6 mg/ml BSA-V, sodium pyruvate (0.25 µM), heparin (5.0 µg/ml), D-penicillamine (20 µM), hypotaurin (10 µM) and epinephrine (1 µM). After one hour of incubation at 39°C, the upper ¾ of the medium were aspirated and the concentration was determined, by means of a haemocytometer, after the swim-up. The concentration of the recovered sperm cells was adjusted to 1 x 10⁴ sperm cells/ml right before the addition of the oocytes (120 µl per 6 oocytes or 40-µl drops per area). The sperm cells and the areas were jointly incubated for 4 h at 39°C.

### Evaluation of the sperm cells bound to the intact area

Following the co-incubation, the oocytes were transferred to PBS supplemented with Pluronic F-68 and pipetted 10 times with a pipette with a 0.5-0.6 mm inner diameter, in order to remove the bound sperm cells. The area-sperm cell complexes were fixated with 2.5% glutaraldehyde for 10 minutes and washed in PBS, stained with 10 µg/ml Hoechst 33342 for 10 minutes and washed again with PBS. The area-sperm cell complexes were placed in carriers and covered. The total count of bound sperm cells was performed with a Nikon Labophot-2 (x200) fluorescence microscope equipped with an ultraviolet UV-1A filter.

**Table 3. Mean number of spermatozoa from studs with a high (Hi), medium (Me) and low (Lo) level of fertility frozen in 3 different diluents, A, B and C, bound to the intact area.**

| Fertility of the male horse | Number of intact areas | Number of spermatozoa bound to the intact area (Mean ± SEM) | | |
|---|---|---|---|---|
| | | A) German diluent* | B) Diluent with HA + LDL* | C) Diluent with LDL* |
| Hi | 12 | 59.17 ± 5.2 | 51.0 ± 5.8^{a} | 39.24 ± 3.8^{a} |
| Me | 14 | 56.03 ± 4.8 | 60.10 ± 6.2^{a} | 76.76 11± 4.1^{b} |
| Lo | 11 | 53.15 ± 5.0 | 91.33 ± 4.8^{b} | 40.34 ± 5.7^{a} |

| | | | | |
|---|---|---|---|---|
| P < 0.05 *A-The German diluent contains intact egg yolk (control); B-homogenate of low-density lipids and high molecular weight hyaluronan (HA + LDL); and C-homogenised lipids alone as a substitute for the egg yolk lipids. | | | | |

### Reindeer semen freezing

The preliminary results of the freezing of equine semen (Reindeer) in a diluent containing 10% LDL lipids and 0.5 mg/ml of high molecular weight HA produced a 60% fertilisation rate, as opposed to the 40% rate obtained using a diluent with egg yolk.

## Claims

1. Method of preparing unilamellar vesicles for the cryopreservation and culture of germ cells and embryos, specially conceived for the preparation of sperm diluents and *in vitro* culture media for embryos of different mammal species, both domestic and wild, also including humans, **characterised in that** it consists of making a presolution of lipids, preferably of vegetable origin, which contain at least 10% of phosphatidylcholine (PCH), such that a suitable quantity of lipids is mixed with a small amount of pure water, heated and constantly stirred; the suspension thus obtained is kept at 50-60°C for the next hour and periodically stirred; subsequently, high molecular weight hyaluronan is added to said lipidic suspension until a final concentration of approximately 1 mg/ml is achieved, which is emulsified by sonication; the lipidic suspension is then pumped and immediately made to go through a membrane, such that, in the final suspension produced, 70% of the particles (SUV) in suspension are within the range of 5 to 15 nm.

2. Method of preparing unilamellar vesicles for the cryopreservation and culture of germ cells and embryos, as claimed in claim 1, **characterised in that** the quantity of vegetable lipids mixed with the hot water is between 4 and 12 mg/ml.

3. Method of preparing unilamellar vesicles for the cryopreservation and culture of germ cells and embryos, as claimed in the preceding claims, **characterised in that** the pure water is heated to 50-60°C and constantly stirred for 30 minutes with a stirrer, for example a magnetic stirrer.

4. Method of preparing unilamellar vesicles for the cryopreservation and culture of germ cells and embryos, as claimed in the preceding claims, **characterised in that** the mixture of lipids and hot water is stirred for 2 minutes every 20 minutes.

5. Method of preparing unilamellar vesicles for the cryopreservation and culture of germ cells and embryos, as claimed in the preceding claims, **characterised in that** the hyaluronan used in said method has a high molecular weight, between 0.3 and 2 mg/ml.

6. Method of preparing unilamellar vesicles for the cryopreservation and culture of germ cells and embryos, as claimed in the preceding claims, **characterised in that** the sonication process is performed in the following manner: at 10 seconds per pulse every 10 seconds with a pulse intensity of 30 units for 5 minutes.

7. Method of preparing unilamellar vesicles for the cryopreservation and culture of germ cells and embryos, as claimed in the preceding claims, **characterised in that** the lipidic suspension is pumped with a homogenising valve at 20,000 psi.

8. Method of preparing unilamellar vesicles for the cryopreservation and culture of germ cells and embryos, as claimed in the preceding claims, **characterised in that** the membrane wherethrough the lipidic suspension is made to pass is preferably a polycarbonate membrane with a 0.05-µm diameter.
